(19) Europäisches Patentamt
European Patent Office
Office européen des brevets

(11) Publication number: 0 116 444
A2

(12) EUROPEAN PATENT APPLICATION

(21) Application number: 84300672.7

(22) Date of filing: 03.02.84

(51) Int. Cl.³: C 07 D 223/16
C 07 D 223/14, C 07 D 223/32
C 07 D 491/04, A 61 K 31/55
//C07C21/24, C07C57/30,
C07C103/78

(30) Priority: 04.02.83 US 463797

(43) Date of publication of application:
22.08.84 Bulletin 84/34

(84) Designated Contracting States:
AT BE CH DE FR GB IT LI LU NL SE

(71) Applicant: ELI LILLY AND COMPANY
307, East McCarty Street
Indianapolis Indiana 46285(US)

(72) Inventor: Paget, Charles Johnson, Jr.
1628 Ridge Hill Avenue
Indianapolis Indiana 46217(US)

(72) Inventor: Ruffolo, Robert Richard, Jr.
9903 Carefree Drive
Indianapolis Indiana 46256(US)

(74) Representative: Crowther, Terence Roger et al,
Erl Wood Manor
Windlesham Surrey GU20 6PH(GB)

(54) **Improvements in or relating to benzazepine derivatives.**

(57) Amino-1H-3-benzazepine derivatives, useful as anti-hypertensive agents, are described herein. These derivatives are prepared by conventional methods, such as heating the corresponding 2-thione or 2-alkoxy compound with an amine, alkylating the corresponding 2-amino compound or de-etherifying the corresponding 6, 7, 8 or 9-alkoxy compound.

EP 0 116 444 A2

Croydon Printing Company Ltd.

# IMPROVEMENTS IN OR RELATING TO BENZAZEPINE DERIVATIVES

This invention concerns a class of novel benzazepine derivatives, which have been discovered to be effective antihypertensive agents.

It has been estimated that 23 million adult Americans have high blood pressure (Hypertension Mechanisms, Diagnosis & Management, J. O. Davis, J. H. Laragh, and A. Selwyn, editors, H. P. Publishing Company, Inc., New York, New York, 1977 p. xi), which is now known to be an underlying cause of a variety of cardiovascular disorders such as heart and kidney diseases as well as stroke. While many drugs are currently available to control hypertension, as a class, the antihypertensive agents are particularly prone to produce side effects which at times are severe enough to limit their utility. For example, the vasodilator, hydralazine, which has become increasingly popular during the past several years to treat hypertension and chronic heart failure, will produce a significant increase in heart rate (reflex tachycardia) in many patients which limits the utility of this drug in those individuals with angina or other underlying heart disease. In addition, higher doses of hydralazine have been associated with a lupus-like syndrome in susceptible individuals. As a result of the excessive tachycardia commonly observed with hydralazine, other drugs,

such as propranolol, are generally used in combination with hydralazine to prevent the increase in heart rate. Therefore, combination therapy with vasodilators has become the norm. Since vasodilators are particularly effective in lowering blood pressure and therefore represent a major class of antihypertensive agents, it is desirable to develop a vasodilating agent which lacks the tachycardia which is characteristic of this class.

Certain related benzazepines are found in the literature. For instance, U.S. Patent No. 3,205,222 describes the preparation and use of 2-amino-7[sic,4]-halobenzazepines which are said to be cockroach poisons and inhibitors of plum curculio. Similar compounds are found in Bull. Soc. Chim. France, 2, 600 (1968), Dissertation Abstracts, 19, 2475 (1959), and J. Het. Chem., 2, 26 (1965). This latter reference also describes the hydrogenation of 2-amino-4-bromo-1H-3-benzazepine to 2-amino-1H-3-benzazepine. No utility is disclosed for this compound. Helvetica Chimica Acta, 64, 373 (1981) describes the preparation of 2-amino-5-phenyl-1H-3-benzazepines. Certain 2-aminobenzazepines were implied as intermediates in the preparation of triazolobenzazepines as described in U.S. Patent 4,022,638 (also U.K. Patent 1,453,910, German Patent 2,442,987, and Chemical Abstracts 83:58833d).

This invention concerns the discovery that certain 2-amino-1H-3-benzazepines are useful antihypertensive agents which produce little or no reflex tachycardia in most animal models. The benzazepines herein disclosed have been shown to possess two activities: (1) they are vasodilators pharmacologically related to hydralazine, and (2) they have the ability to decrease heart rate directly (bradycardic activity) at doses similar to those required for the vasodilating activity. These two activities provide for a unique profile of action which has not been demonstrated previously. Animal studies indicate that the antihypertensive activity of the present benzazepines results from their vasodilating activity while the bradycardic activity of these compounds serves to attenuate or abolish the reflex tachycardia which would normally result from vasodilitation and the subsequent decrease in blood pressure, as in the case for hydralazine.

This invention concerns compounds of the formula

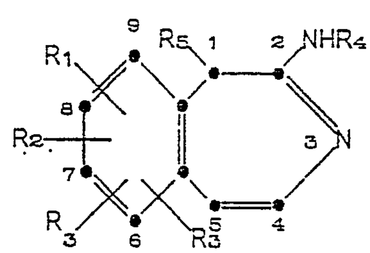

I

and pharmaceutically acceptable salts thereof, wherein

each $R_1$ and $R_2$ is independently hydrogen, hydroxy, halo, $C_1$-$C_3$ alkyl, nitro, amino, mono- or di-$C_1$-$C_3$ alkylamino, trifluoromethyl, nitrile, amide, acetyl, or $C_1$-$C_3$ alkoxy, or when taken together form a methylenedioxy group or when taken together with the benzene ring to which they are attached form a naphthalene, tetrahydronaphthalene, dihydrobenzopyran, dihydrobenzofuran, or indan system;

each $R_3$ is independently hydrogen, halo, $C_1$-$C_3$ alkyl, or $C_1$-$C_3$ alkoxy;

$R_4$ is hydrogen, $C_1$-$C_4$ alkyl, allyl, propargyl, or cyclopropyl;

$R_5$ is hydrogen or methyl;

with the proviso that $R_1$, $R_2$, $R_3$, $R_4$, and $R_5$ may not all be hydrogen at the same time, and with the further proviso that when both $R_3$ are hydrogen and one of $R_1$ and $R_2$ is hydroxy, the other of $R_1$ and $R_2$ may not be hydrogen or hydroxy.

A preferred group of compounds of formula I above is that wherein each $R_1$ and $R_2$ is independently hydrogen, hydroxy, halo, $C_1$-$C_3$ alkyl, nitro, amino, mono- or di-$C_1$-$C_3$ alkylamino, trifluoromethyl, or $C_1$-$C_3$ alkoxy, or when taken together form a methylenedioxy group or when taken together with the benzene ring to which they are attached form a naphthalene, tetrahydronaphthalene, or indan system; one $R_3$ is hydrogen and the other $R_3$ is hydrogen, $C_1$-$C_3$ alkyl, or $C_1$-$C_3$ alkoxy; $R_4$ is hydrogen, $C_1$-$C_3$ alkyl, allyl, propargyl or cyclopropyl; and $R_5$ is defined as before.

The compounds of formula I above are prepared by:

(a) heating a compound of the formula

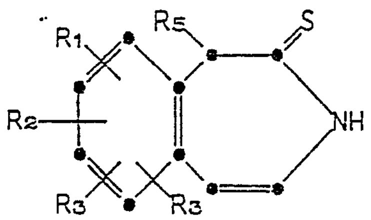

V

wherein $R_1$, $R_2$, $R_3$ and $R_5$ are defined as above, with $R_4NH_2$ or its salt wherein $R_4$ as defined above, optionally in the presence of an alcohol or acetonitrile; or

(b) heating a compound of the formula

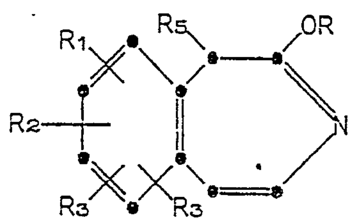

VI

wherein $R_1$, $R_2$, $R_3$ and $R_5$ are as defined above and R is $C_1$-$C_6$ alkyl, with an acid salt of $R_4NH_2$ where $R_4$ is defined as above, in a non-reactive solvent; or

(c) reacting a compound of the formula

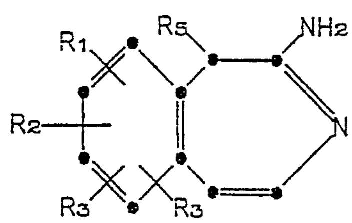

Ia

wherein $R_1$, $R_2$, $R_3$ and $R_5$ are defined as above, with $R_4'NH_2$ salt wherein $R_4'$ is $C_1$-$C_4$ alkyl, allyl, propargyl, or cyclopropyl, in an alcohol; or

(d) de-etherifying a compound of the formula

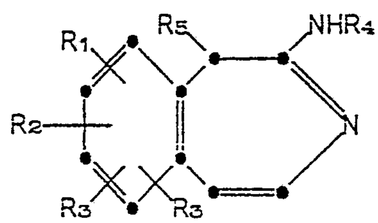

Ib

wherein $R_4$ and $R_5$ are defined as above, and at least one of $R_1$, $R_2$ and $R_3$ is $C_1$-$C_3$ alkoxy, with methanesulfonic acid/methionine, ethanethiol/aluminum chloride in the presence of 1,2-dichloroethane, $PBr_3$ in a halogenated hydrocarbon solvent, or HBr in glacial acetic acid; or

(e) nitrating a compound of the formula

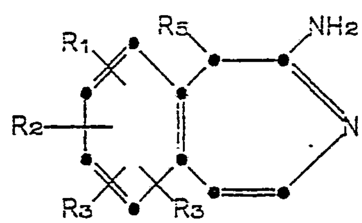

Ic

wherein $R_3$ and $R_5$ are defined as above, and one of $R_1$ and $R_2$ is hydrogen, with nitric acid;

optionally reducing the $NO_2$ group to provide the compounds of formula I wherein one of $R_1$ and $R_2$ is amino; and

optionally alkylating the $NH_2$ group of $R_1$ or $R_2$ to provide the compounds of formula I wherein one of $R_1$ and $R_2$ is mono- or di-$C_1$-$C_3$ alkylamino; or

(f) cleaving a compound of the formula

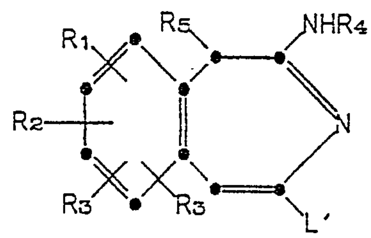

Id

wherein $R_4$ and $R_5$ are defined as above, and L' is a leaving group, with a hydrogenation agent; or

(g) alkylating a compound of the formula

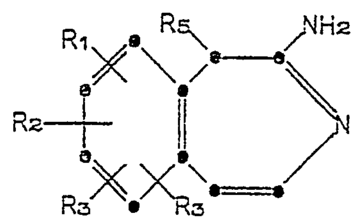

Ie

wherein $R_3$ and $R_5$ are defined as above, one or both of $R_1$ and $R_2$ are hydroxy, with alkylating agent, optionally in the presence of a base; or

(h) cyclizing a compound of the formula

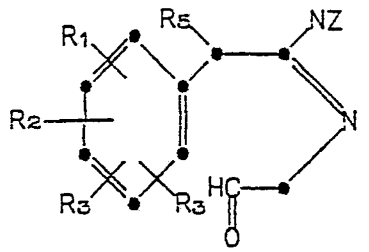

If

wherein $R_1$, $R_2$, $R_3$ and $R_5$ are defined as above, and Z is a protecting group, with an acid; or

(i) cleaving a protecting group from a compound of the formula

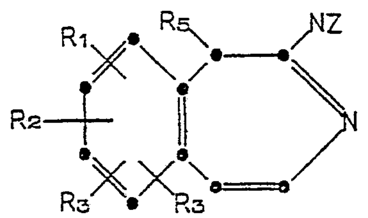

Ig

wherein $R_1$, $R_2$, $R_3$ and $R_5$ are defined as above, Z is a protecting group, with acid, base, a hydrogenation agent, or water and $PdCl_2$; or

(j) reacting a compound of the formula

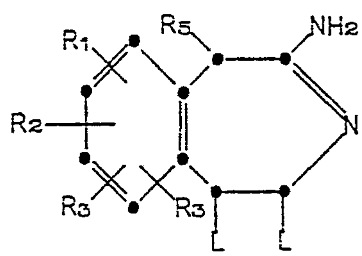

Ih

wherein $R_1$, $R_2$, $R_3$ and $R_5$ are defined as above, and at least one L is hydrogen and the other L is hydrogen or a leaving group, with, when L is other than hydrogen, an acid catalyst, at reflux temperature, or with, when both L terms are hydrogen, dichlorodicyanobenzoquinone, $MnO_2$ or Pd, to introduce a $\Delta^4$ double bond; or

(k) cleaving a compound of the formula

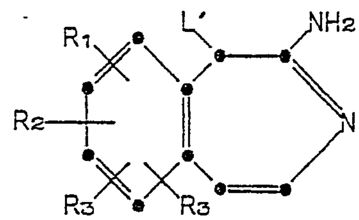

Ii

where $R_1$, $R_2$ and $R_3$ are defined as above, and L' is a leaving group, with a hydrogenating agent, in the presence of a base; or

(l) optionally salifying a product of any of the preceding steps by conventional methods.

Also within the scope of the present invention are novel intermediate compounds of formula VII below that are used to prepare the compounds of formula I above. These intermediates are of the formula

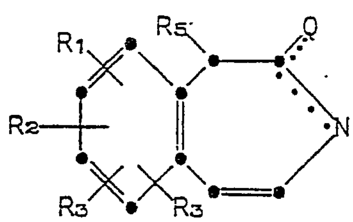

VII

wherein $R_1$, $R_2$, $R_3$, and $R_5$ are as defined above for formula I, Q is halo, -O-($C_1$-$C_3$ alkyl), -S-($C_1$-$C_3$ alkyl), -SO-($C_1$-$C_3$ alkyl), -$SO_2$-($C_1$-$C_3$ alkyl), =O or =S, and the dotted line represents the presence of a double bond which is only exo to the ring when Q is =S or =O, or salts thereof.

Especially preferred embodiments of formula VII are those compounds where Q is methoxy or ethoxy (shown by formula VI above), where Q is =S (shown by formula V above) and where Q is =O (shown by formula IV above).

A further aspect of this invention is a pharmaceutical formulation comprising as active ingredient one or more of the benzazepine derivatives of formula I, or a pharmaceutically-acceptable salt thereof, associated with one or more pharmaceutically-acceptable carriers, diluents, or excipients therefor. The formulations are particularly useful in treating mammals suffering from hypertension.

An additional embodiment of this invention is a method of reducing blood pressure in a mammal with elevated blood pressure and in need of treatment which comprises administering to the mammal a hypotensive dose of a benzazepine derivative of formula I, or a pharmaceutically-acceptable salt thereof.

The term "$C_1$-$C_6$ alkyl" refers to the straight and branched aliphatic radicals of one to six carbon atoms such as methyl, ethyl, isopropyl, n-propyl, n-butyl, tert-butyl, pentyl, isopentyl, hexyl, and the like, and includes within its definition the term

"$C_1$-$C_3$ alkyl". The term "halo" refers to fluoro, chloro, bromo and iodo. As will be understood by those skilled in the art, when $R_1$ and $R_2$ taken together form a methylenedioxy group or a naphthalene, tetrahydronaphthalene, dihydrobenzopyran, dihydrobenzofuran, or indan system as previously described, the substitution on the aromatic ring will necessarily be at adjacent positions.

An especially preferred group of compounds of formula I are those wherein:

a) one of $R_1$, $R_2$ and $R_3$ is $C_1$-$C_3$ alkoxy, especially methoxy;

b) two of $R_1$, $R_2$, and $R_3$ are each $C_1$-$C_3$ alkoxy, especially methoxy;

c) one of $R_1$, $R_2$, and $R_3$ is $C_1$-$C_3$ alkyl, especially methyl;

d) two of $R_1$, $R_2$, and $R_3$ are each $C_1$-$C_3$ alkyl, especially methyl;

e) one of $R_1$, $R_2$, and $R_3$ is $C_1$-$C_3$ alkoxy, especially methoxy, and another of $R_1$, $R_2$, and $R_3$ is $C_1$-$C_3$ alkyl, especially methyl;

f) two of $R_1$, $R_2$, and $R_3$ are at the 7 and 8 positions of the ring system;

g) $R_4$ is hydrogen; and

h) $R_5$ is hydrogen.

Particularly preferred compounds of formula I are 2-amino-7,8-dimethoxy-1H-3-benzazepine and 2-amino-7,8-dimethyl-1H-3-benzazepine.

A preferred formulation comprises the compound 2-amino-7,8-dimethoxy-1H-3-benzazepine or a pharmaceutically acceptable salt thereof, especially the hydrochloride salt, in combination with a pharmaceutical carrier therefor. An especially preferred formulation is one intended for oral use.

A preferred method of treatment comprises administering a dose effective for lowering blood pressure of the compound 2-amino-7,8-dimethoxy-1H-3-benzazepine or a pharmaceutically acceptable salt thereof, especially the hydrochloride salt.

The compounds of formula I may be prepared by methods known in the art. One method is described by Johnson and Nasutavicus, J. Het. Chem., 2, 26 (1965). According to this method, o-phenylenediacetonitrile may be cyclized to 2-amino-4-bromo-1H-3-benzazepine in the presence of hydrogen bromide and acetic acid, which upon hydrogenation gives 2-amino-1H-3-benzazepine. The compounds of formula I may be prepared in an analogous manner, starting from the appropriately substituted o-phenylenediacetonitriles, which may be prepared by reacting cyanide ion with the respective 1,2-di(halomethyl)benzenes. The di(halomethyl)benzenes, otherwise known as $\alpha,\alpha'$-dihalo-o-xylenes, may be prepared by haloalkylation of benzenes, free radical halogenation of o-xylenes, and similar methods known in the art.

An alternate and preferred method of preparing the compounds of formula I involves the reaction of a phenylacetic acid (II) according to the following scheme:

Scheme I

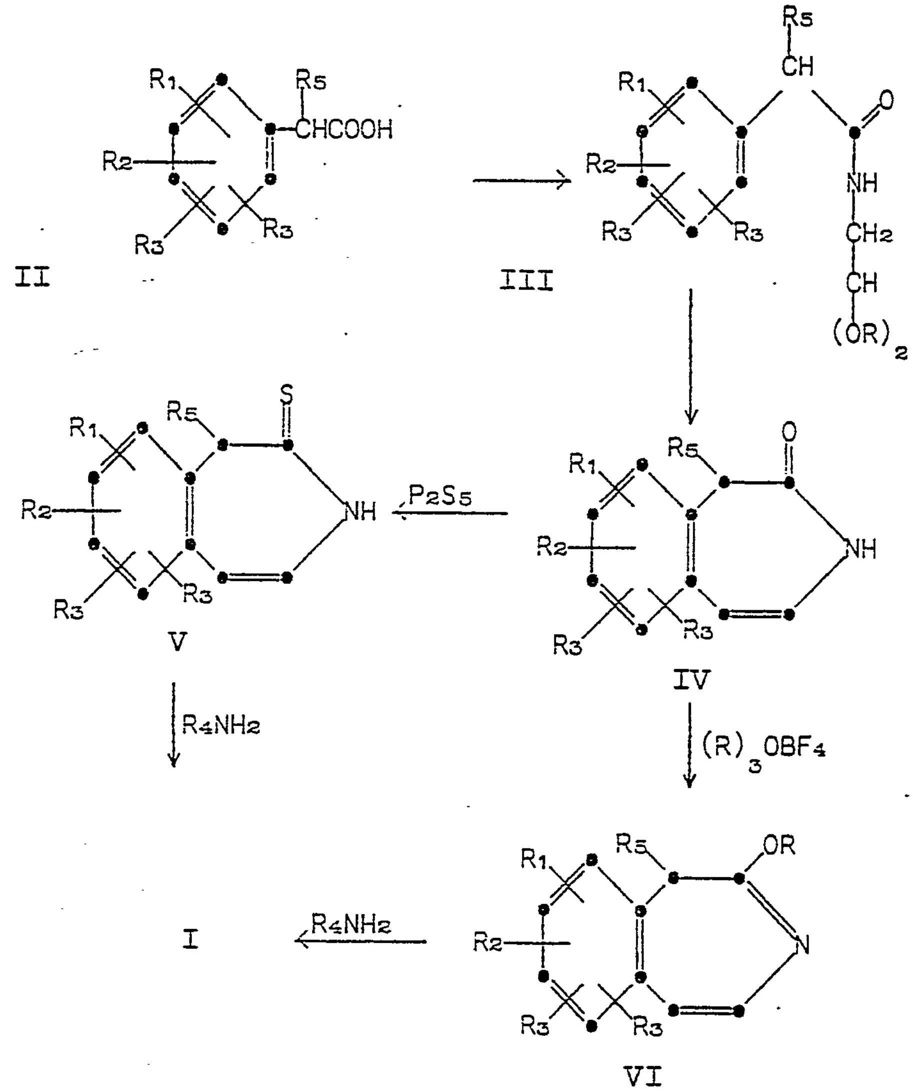


where $R_1$, $R_2$, $R_3$, $R_4$, and $R_5$ are the same as previously described and R is $C_1$-$C_6$ alkyl.

The phenylacetic acid (II) is converted to the dialkoxyethyl amide III according to any of several known transformations of carboxylic acids into carboxamides. For example, the acid (II) may be first converted into an acid halide, preferably the acid chloride, by treatment with a halogenating agent, preferably thionyl chloride or oxalyl chloride, usually in the presence of a weak base, such as pyridine, and optionally in the presence of an inert solvent, such as benzene, xylene, dioxane, and the like. The acid halide may be first isolated or may be used in situ. The acid halide is then allowed to react with an aminoacetaldehyde dialkyl acetal in the presence of a weak base, such as sodium carbonate, and optionally in an inert solvent, such as acetonitrile.

An alternate method of preparing compounds of formula III involves the reaction of the phenylacetic acid II with an aminoacetaldehyde dialkyl acetal in the presence of a dehydrating agent such as 1,1'-carbonyldiimidazole.

The intermediate amide III can be cyclized to the lactam IV in the presence of any of a number of acidic cyclization reagents, especially polyphosphoric acid, 48% aqueous hydrobromic acid, methanesulfonic acid (alone or in combination with phosphorous pentoxide), sulfuric acid, hydrogen chloride in acetic acid, or phosphoric acid. The cyclization occurs at temperatures from about ambient temperature to about 100°C.

Lactam IV can be transformed into the thiolactam V by heating with phosphorous pentasulfide and a trialkylamine, such as triethylamine, in a non-reactive solvent such as pyridine at temperatures from about 40°C. to about 100°C. Thiolactam V can then be transformed into the desired product I by heating in the presence of the appropriate amine $R_4NH_2$ or its salt, optionally in the presence of an alcohol. Temperatures of about 40°C. up to the reflux temperature of the mixtures are preferred.

Alternatively, lactam IV may be first converted to the corresponding 2-ethoxy- or 2-methoxy-1H-3-benzazepines VI by treating the intermediate IV with triethyl- or trimethyloxonium tetrafluoroborate, respectively, in methylene chloride. Typically, the reaction is complete after stirring for about three days at room temperature. Heating the resulting 2-alkoxy-1H-3-benzazepine VI with an acid salt, preferably the acetate, of $R_4NH_2$ in a non-reactive solvent such as an alcohol gives the desired 2-amino compound I. Reaction temperatures of about 40°C. to the reflux temperature of the mixtures are employed. Under the latter condition, the reaction is usually complete within two hours.

The compounds of formula I where $R_4$ is other than hydrogen may be formed directly by treating the thiolactam intermediate V with the appropriate amine $R_4'NH_2$ ($R_4'$ is $C_1$-$C_4$ alkyl, allyl, propargyl, or cyclopropyl) usually in the presence of an alcohol, such as

methanol or ethanol, or acetonitrile. The substituted amine derivative may also be obtained directly from the 2-ethoxy or 2-methoxy intermediate VI formed from the lactam compound IV by stirring the alkoxy intermediate in an alcohol solution of the acetate salt of $R_4'NH_2$. The substituted amine compounds of formula I may also be formed from the preferred unsubstituted compounds (formula I, $R_4$ is hydrogen) by stirring with a $R_4'NH_2$ salt in an alcohol at temperatures from about ambient temperature up to the reflux temperature of the mixture.

The 1-methyl compounds of formula I may be prepared from the corresponding 1-(1-haloethyl)-2-(halomethyl)benzenes by the procedure of Johnson and Nasutavicus or from the α-methylphenyl acetic acid derivatives following Scheme I. A preferred method, however, is by alkylating one of the intermediates of Scheme I where $R_5$ is hydrogen, preferably a 2-ethoxy- or 2-methoxy-1H-3-benzazepine compound, by first treating with a strong base, such as lithium diisopropyl amide, followed by treatment with a methyl halide. The reaction of the intermediate and the strong base is preferably carried out at -20 to -78°C. in a non-reactive solvent, such as tetrahydrofuran. The 1-methyl compound of formula I may then be prepared from the corresponding 1-methyl intermediate according to the same procedures as previously described.

Certain compounds of formula I may be prepared by the transformation of other compounds of formula I. For example, 2-amino-7,8-dihydroxy-1H-3-benzazepine or a suitable precursor thereof, may be prepared from the respective 7,8-dimethoxy congener by treating with boron tribromide in 1,2-dichloroethane.

This dihydroxy intermediate may then be dialkylated with the appropriate alkyl halide in the usual way to give the corresponding 7,8-dialkoxy derivative. Other alkoxy compounds may be similarly prepared. Other cleavage reactions which may be employed include methanesulfonic acid/methionine and ethanethiol/aluminum chloride in the presence of 1,2-dichloroethane. In the case of the 7,8-dimethoxy compound of formula I, the former reaction results in selective ether cleavage to give the 7-hydroxy-8-methoxy compound while the latter reaction provides the 7-methoxy-8-hydroxy derivative. This also provides an alternate preparation of dissimilar alkoxy substituted compounds of formula I. When two or more alkoxy groups are present they are all converted to the corresponding hydroxy compound by cleavage using known reagents such as $PBr_3$ in a halogenated hydrocarbon solvent, such as dichloromethane or HBr in glacial acetic acid.

Other compounds of formula I may be formed by transformations such as nitration. For instance, 2-amino-1H-3-benzazepine may be nitrated to give the 8-nitro derivative which may then be reduced to the corresponding amine compound. An amine compound may be alkylated to give the mono- or di-alkyl amine substituents on the aromatic ring. The amine functionalities may also be introduced by heating the corresponding halo derivative of formula I with the appropriate amine in a high-boiling non-reactive solvent to provide the corresponding amino and mono- and di-alkylamino compounds of formula I.

Other methods which can be used to form the compounds of formula I use a cyclization of an aldehyde of formula If above with an acid having the 2-amino group protected. The cyclization occurs in one step with concomment removal of the protecting group, if needed. Examples of a protecting group would be $R_4'$, defined above, trimethylsilane, or other silyl functions. Suitable acids are mineral acids, such as sulfuric acid or hydrochloric acid, organic acids, such as acetic acid or propionic acid, or any acid customarily used in acid catalysis.

The compounds of formula Ig above are converted to the compounds of formula I by removal of the protecting group Z. Examples of such protecting groups and their removal are well known in the art. Some of these groups are described above for formula If. When Z is $NH_2$ or NHZ' where Z' is a protecting moiety, the corresponding primary amine is formed by hydrogenation. When Z is phthalic anhydride, the corresponding primary amine is formed by hydrolysis under either acidic or basic conditions. When Z is $-CO(C_1-C_3$ alkyl), -CO-phenyl, $-CO-CF_3$, benzyl, -CH(phenyl)(p-methoxyphenyl), or $-CH_2-CH=CH_2$, the primary amine is formed by hydrolysis under either acidic or basic conditions, hydrogenation under standard conditions, or water and $PdCl_2$ when Z is $-CH_2-CH=CH_2$. When Z is OH, $-O-(C_1-C_3$ alkyl), or $-CO-(C_1-C_3$ alkyl), then $H_2/Pd$, $H_2/Pt$ or $LiAlH_4$ is used under standard reaction conditions.

Introducing the $\Delta^4$ double bond in a compound of formula Ih above can be done by a variety of methods. When the L term is hydrogen, the double bond is formed by using dichlorodicyanobenzoquinone (DDQ), $MnO_2$, or Pd in a solvent such as dioxane, hydrocarbons e.g. hexane, or halogenated solvents, e.g. dichloromethane, at reflux. When L is a leaving group other than hydrogen, L is cleaved to form the double bond using a polar solvent, with an acid catalyst, at reflux temperature. The compound of formula Ih can be formed by cyclization methods known in the literature and is formed in situ in the reaction mixture or is isolated therefrom if desired. Examples of L moieties under the above conditions are: hydrogen; halogen; $-V-C_1-C_3$ alkyl, $-V-\underset{\underset{O}{\|}}{C}-(C_1-C_3$ alkyl), where V is O, S, N, P or $SO_2$; trialkylamine; or $-O-\underset{\underset{S}{\|}}{C}-SCH_3$. Heat or acid and heat is used to effect the removal of L.

Another cleavage reaction involving a leaving group is described above where the L' term of formula Ii is reacted with a hydrogenating agent, e.g. $H_2/Pd$, $H_2/Pt$, under standard conditions, in the presence of a base such as $NaHCO_3$ or $K_2CO_3$. The L' moiety is defined as L above, but excluding hydrogen.

The compounds of formula Id above are reacted by hydrogenation conditions as described for the reaction of the compounds of formula Ii above. L' is defined as for formula Ii above.

The pharmaceutically acceptable acid addition salts of formula I include salts derived from inorganic acids such as hydrochloric acid, nitric acid, phosphoric acid, sulfuric acid, hydrobromic acid, hydroiodic acid, phosphorous acid and the like, as well as salts derived from nontoxic organic acids such as aliphatic mono and dicarboxylic acids, phenyl-substituted alkanoic acids, hydroxy alkanoic and alkandioic acids, aromatic acids, aliphatic and aromatic sulfonic acids, etc. Such pharmaceutically acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, malonate, succinate, suberate, sebacate, fumarate, maleate, mandelate, butyne-1,4-dioate, hexyne-1,6-dioate, benzoate, chlorobenzoate, methylbenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycollate, malate, tartrate, methanesulfonate, propanesulfonate, naphthalene-1-sulfonate, naphthalene-2-sulfonate, and the like salts. The preferred salt form is the hydrochloric acid salt.

The compounds of formula I may be administered by any number of routes, including the oral, subcutaneous, intramuscular, intravenous, transdermal, and

rectal routes, usually employed in the form of a pharmaceutical composition, although it is a special feature of the compounds of formula I that they are effective when administered orally. Such compositions are prepared in a manner well known in the pharmaceutical art and comprise one or more active compounds of formula I.

Accordingly, the invention includes a pharmaceutical composition comprising as active ingredient a compound of formula I, or an acid addition salt thereof, associated with a pharmaceutically acceptable carrier. In making the compositions of the present invention, the active ingredient will usually be mixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus the composition can be in the form of tablets, pills, powders, lozenges, sachets, cachets, elixirs, emulsions, solutions, syrups, suspensions, aerosols (as a solid or in a liquid medium), ointments containing for example up to 10% by weight of the active compound, soft and hard gelatin capsules, suppositories, sterile injection solutions, and sterile packaged powders.

Some examples of suitable carriers are lactose, dextrose, sucrose, sorbitol, mannitol, starches, gum acacia, calcium phosphate, alginates, calcium

silicate, microcrystalline cellulose, polyvinylpyrrolidone, cellulose, tragacanth, gelatin, syrup, methyl cellulose, methyl- and propylhydroxybenzoates, talc, magnesium stearate, water, or mineral oil. Either solid or liquid formulations can include lubricating agents, wetting agents, emulsifying and suspending agents, preserving agents, sweetening agents or flavoring agents. The present compositions may, as is well known in the art, be formulated so as to provide quick, sustained or delayed release of the active ingredient of formula I after administration to the patient.

For oral administration, which is preferred, a compound of formula I is admixed with carriers and diluents and molded into tablets or enclosed in gelatin capsules. The mixtures can alternatively be dissolved in liquids such as ten percent aqueous glucose solution, isotonic saline, sterile water, or the like, and administered intravenously or by injection. Such solutions can, if desired, be lyophilized and stored in a sterile ampoule ready for reconstitution by the addition of sterile water for ready intramuscular injection.

Preferably the compositions are formulated in a unit dosage form, each dosage containing from 5 to 500 mg., more usually 25 to 300 mg., of the active ingredient of formula I. The term "unit dosage form" refers to physically discrete units suitable as unitary dosages for human subjects and animals, each unit containing a predetermined quantity of active material calculated to produce the desired therapeutic effect, in association with the required pharmaceutical carrier.

The active compounds of formula I are effective over a wide dosage range. For example, dosages per day will normally fall within the range of 0.5 to 300 mg./kg. In the treatment of adult humans, the range of about 1 to 50 mg./kg., in single or divided doses, is preferred. However it will be understood that the amount of the compound actually administered will be determined by a physician, in the light of the relevant circumstances including the condition to be treated, the choice of compound to be administered, the age, weight, and response of the individual patient, the severity of the patient's symptoms, and the chosen route of administration, and therefore the above dosage ranges are not intended to limit the scope of the invention in any way.

Although the compounds of formula I are effective antihypertensive agents by themselves, they may also be administered in combination with one or more other antihypertensive and/or diuretic agents if desired. Such therapy may be provided by administering the agents as individual dosage units or as a single dosage unit comprising a mixture of the agents. Such additional agents include clonidine, methyldopa, hydralazine, diazoxide, amiloride, nifedipine, propranolol, reserpine, timolol, cyclothiazide, hydrochlorothiazide, indacrinone, and the like.

In an effort to illustrate more fully the present invention, the following detailed examples are provided. The examples are illustrative only and are not intended to limit the scope of the invention.

Example 1

2-Amino-7,8-diethyl-1H-3-benzazepine hydrochloride

A. Preparation of 3,4-diethylbenzyl chloride.

Twenty-five grams of *o*-diethylbenzene were dissolved in acetic acid. Approximately 1 g. of zinc chloride and 18 g. of paraformaldehyde were added. Hydrogen chloride gas was bubbled through the solution and the solution was heated at 55°C. for three hours. Orthophosphoric acid was added and the solution was heated to 75°C. for six hours with hydrogen chloride gas continuing to be bubbled through the solution. The solution was then poured into water and was extracted with diethyl ether. The ether was washed first with water and then with a saturated sodium carbonate solution. The ether was dried and evaporated *in vacuo* to give 30 g. of 3,4-diethylbenzyl chloride as an oil.

B. Preparation of 3,4-diethylphenylacetic acid.

Thirty grams of 3,4-diethylbenzyl chloride in dimethylsulfoxide were reacted with 9.8 g. of sodium

cyanide. After stirring the reaction overnight at room temperature, the reaction was poured into water and extracted with diethyl ether. The ether was dried over magnesium sulfate, filtered, and evaporated in vacuo to give 17 g. of an oil. The oil was dissolved in 100 ml. of ethanol and 100 ml. of 10 N sodium hydroxide was added. The solution was refluxed overnight. The ethanol was evaporated in vacuo. More water was added to the solution and the solution was extracted with diethyl ether. The water layer was made acidic with hydrochloric acid. The acidic solution was extracted with methylene chloride. The methylene chloride solution was dried and evaporated in vacuo to yield 14.5 g. of 3,4-diethylphenylacetic acid as an oil.

C. Preparation of N-(2,2-dimethoxyethyl)-3,4-diethylphenylacetamide.

To a solution of 14.5 g. of 3,4-diethylphenylacetic acid in methylene chloride were added 12.32 g. of 1,1'-carbonyldiimidazole. After stirring for 15 minutes, 8.3 ml. of aminoacetaldehyde dimethyl acetal were added and the reaction was stirred for an additional 30 minutes. The solution was washed with a saturated solution of sodium bicarbonate followed by several washings with water. The organic solution was dried over magnesium sulfate and evaporated in vacuo to give 18 g. of N-(2,2-dimethoxyethyl)-3,4-diethylphenylacetamide as an oil.

D. Preparation of 1,3-dihydro-7,8-diethyl-2H-3-benzazepine-2-one.

Eighteen grams of N-(2,2-dimethoxyethyl)-3,4-diethylphenylacetamide were heated in methanesulfonic acid overnight on a steam bath giving an internal temperature of 70-80°C. The solution was poured onto ice which resulted in the formation of a solid precipitate. The precipitate was collected by filtration and purified by gradual precipitation from methanol/ethyl acetate/diethyl ether resulting in 1.79 g. of 1,3-dihydro-7,8-diethyl-2H-3-benzazepine-2-one.

E. Preparation of 2-amino-7,8-diethyl-1H-3-benzazepine hydrochloride.

To a solution of 1.79 g. of 1,3-dihydro-7,8-diethyl-2H-3-benzazepine-2-one in methylene chloride were added 1.23 g. of trimethyloxonium tetrafluoroborate. The reaction was stirred at room temperature for about three days. The solvent was removed by evaporation and the residue was redissolved in methanol. Excess ammonium acetate was added and the solution was refluxed for two hours. After removing the solvent *in vacuo*, water was added and the solution was warmed on a steam plate. The solution was filtered and the filtrate was added to a sodium bicarbonate solution. The resulting precipitate was collected by filtration and dissolved in methanol. Dry hydrogen chloride gas was

bubbled into the solution resulting in a precipitate which was recovered by filtration. The precipitate was crystallized from methanol/ethyl acetate to give 0.186 g. of the title product, m.p. >230°C. with decomposition.

Analysis: $C_{14}H_{18}N_2 \cdot HCl$;
Calc.: C, 67.05; H, 7.64; N, 11.17;
Found: C, 66.82; H, 7.57; N, 10.88.

Example 2

2-Amino-7,8-dimethyl-1H-3-benzazepine hydrochloride

Following the procedures of Examples 1B-1E, 50 g. of 3,4-dimethylbenzyl chloride were transformed into 3.16 g. of the title product, m.p. ·250°C. with decomposition.

Analysis: $C_{12}H_{14}N_2 \cdot HCl$;
Calc.: C, 64.71; H, 6.79; N, 12.58; Cl, 15.92;
Found: C, 64.46; H, 6.79; N, 12.29; Cl, 16.26.

Example 3

2-Amino-1H-naphth[2,3-d]azepine

Ten grams of 2,3-di(cyanomethyl)naphthalene were added to 200 ml. of glacial acetic acid which had previously been saturated with hydrogen bromide gas at 0°C. The reaction was stirred overnight and the resulting suspended solid was recovered by filtration. The solid was triturated with a saturated sodium bicarbonate solution. The insoluble solid was filtered, washed with water and dried. Crystallization from hot N,N-dimethylformamide gave 9.5 g. of 2-amino-4-bromo-1H-naphth[2,3-d]azepine, m.p. about 235-240°C.

This bromo intermediate (8.61 g.) was hydrogenated in the presence of 1.2 g. of 10% palladium-on-carbon and potassium bicarbonate in 1.2 liters of ethanol. Filtration of the reaction solution, evaporation of the solvent, and recrystallization from ethanol afforded 4.29 g. of 2-amino-1H-naphth[2,3-d]-azepine, m.p. about 253-254°C. The NMR and infrared spectra were consistent with the structure of the desired product.

## Example 4

2-Amino-8-methoxy-1H-3-benzazepine hydrochloride

A. Preparation of N-(2,2-diethoxyethyl)-3-methoxyphenylacetamide.

To a solution of 8.3 g. of 3-methoxyphenylacetic acid in chloroform were added 8.1 g. of 1,1'-carbonyldiimidazole. A solution of 6.7 g. of aminoacetaldehyde diethyl acetal in chloroform was slowly added while the reaction mixture was cooled with an external ice bath. The reaction was stirred at room

temperature for about 90 minutes. The reaction solution was then washed three times with water, once with an aqueous sodium carbonate solution, once with a 1N hydrochloric acid solution, and twice more with water. The organic solution was dried over magnesium sulfate, filtered, and evaporated to dryness giving 10.5 g. of N-(2,2-diethoxyethyl)-3-methoxyphenylacetamide as a light oil.

B. Preparation of 1,3-dihydro-8-methoxy-2H-3-benzazepine-2-one.

Forty-six grams of N-(2,2-diethoxyethyl)-3-methoxyphenylacetamide were stirred in about 200 ml. of 48% aqueous hydrobromic acid at room temperature for three hours. The reaction was diluted with water and filtered. The precipitate was crystallized from ethanol affording 15 g. of 1,3-dihydro-8-methoxy-2H-3-benzazepine-2-one.

C. Preparation of 1,3-dihydro-8-methoxy-2H-3-benzazepine-2-thione.

A solution of 43.1 g. of 1,3-dihydro-8-methoxy-2H-3-benzazepine-2-one, 25.4 g. of phosphorous pentasulfide, and 600 ml. of pyridine was heated to 90-100°C. for about one hour followed by heating at 60-90°C. for an additional hour. After cooling, the reaction mixture was poured into 1N hydrochloric acid. The resulting precipitate was washed with acetone and

then crystallized from acetone affording 27 g. of the desired 1,3-dihydro-8-methoxy-2H-3-benzazepine-2-thione, m.p. about 196-197°C.

D. Preparation of 2-amino-8-methoxy-1H-3-benzazepine hydrochloride.

A mixture of 4.7 g. of 1,3-dihydro-8-methoxy-2H-3-benzazepine-2-thione and 50 ml. of anhydrous ammonia was heated to 40°C. in a sealed bomb for three hours. After the ammonia was removed by evaporation, diethyl ether was added and the resulting slurry was filtered. The precipitate was dissolved in about 75 ml. of hot acetic acid and anhydrous hydrogen chloride gas was bubbled into the solution. The solution was diluted with diethyl ether and filtered. The precipitate was dissolved in water and filtered to remove insoluble matter. The filtrate was neutralized with sodium carbonate and the free base of the title product was recovered by filtration. The salt formation was repeated twice again giving 2 g. of the desired title product, m.p. about 250-252°C. with decomposition.

Analysis: $C_{11}H_{12}N_2O\cdot HCl$;

Calc.: C, 58.80; H, 5.83; N, 12.47;

Found: C, 58.72; H, 5.76; N, 12.44.

## Example 5

2-Amino-7,8-dimethoxy-1H-3-benzazepine

A. Preparation of N-(2,2-diethoxyethyl)-3,4-dimethoxyphenylacetamide.

The desired intermediate was prepared from 3,4-dimethoxyphenylacetic acid following the procedure of Example 4A. The following alternate procedure also prepared the desired intermediate. Thirty grams of oxalyl chloride were added to a slurry of 16 g. of 3,4-dimethoxyphenylacetic acid in 250 ml. of benzene. After about 30 minutes, one drop of pyridine was added to the reaction solution and the temperature rose to about 45°C. After an additional half hour, two more drops of pyridine were added. After additional stirring an additional 5 ml. of oxalyl chloride and two drops of pyridine were added. After an additional half hour the solvents were removed in vacuo. The resulting oil was added to a slurry of 17 g. of sodium carbonate and 11 g. of aminoacetaldehyde diethyl acetal in 200 ml. of acetonitrile using an external ice bath to cool the solution. After stirring overnight, the reaction mixture was poured into ice water. The solution was extracted four times with methylene chloride. The combined organic extracts were dried over magnesium sulfate and evaporated in vacuo to yield 25 g. of the desired intermediate as an oil which solidified on standing.

B. Preparation of 1,3-dihydro-7,8-dimethoxy-2H-3-benzazepine-2-one.

Following the procedure of Example 4B, N-(2,2-diethoxyethyl)-3,4-dimethoxyphenylacetamide was cyclized to the desired lactam intermediate. This lactam intermediate could also be formed by treating the phenylacetamide intermediate with phosphoric acid for three hours followed by the same workup.

C. Preparation of 2-ethoxy-7,8-dimethoxy-1H-3-benzazepine.

A slurry of 1.1 g. of 1,3-dihydro-7,8-dimethoxy-2H-3-benzazepine-2-one in 25 ml. of methylene chloride was cooled to 10°C. Under a nitrogen blanket, 1.1 g. of triethyloxonium tetrafluoroborate in about 10 ml. of methylene chloride were added in a dropwise fashion. The reaction mixture was allowed to warm to room temperature and was stirred for about three days under a nitrogen atmosphere. A total of 1.2 g. of triethyloxonium tetrafluoroborate were added and the reaction allowed to stir overnight. Twenty ml. of a saturated aqueous sodium carbonate solution were added and the resulting layers were separated. The organic layer was dried over magnesium sulfate and evaporated in vacuo. The resulting semi-solid oil was slurried in ethyl acetate and filtered. The filtrate was evaporated to give a brown oil which was identified by NMR to be the desired 2-ethoxy intermediate.

D. Preparation of 2-amino-7,8-dimethoxy-1H-3-benzazepine.

A solution of 1.5 g. of 2-ethoxy-7,8-dimethoxy-1H-3-benzazepine and 5 g. of ammonium chloride in 25 ml. of methanol was refluxed under a nitrogen atmosphere overnight. The solvent was evaporated and the residue was taken up in water. The pH of the solution was approximately 5 and the solution was extracted with ethyl acetate. The aqueous solution was brought to pH 9 with sodium carbonate and the aqueous solution was extracted twice with ethyl acetate and once with chloroform. The three extracts at pH 9 were combined and evaporated to dryness giving 0.5 g. of the desired product. m.p. 183-185°C.

Analysis: $C_{12}H_{16}N_2O_2$;
Calc.: C, 66.04; H, 6.47; N, 12.84;
Found: C, 66.28; H, 6.62; N, 13.12.

Example 6

2-Amino-7,8-dimethoxy-1H-3-benzazepine hydrochloride

The title product hydrochloride salt was prepared in an alternate manner from the lactam intermediate of Example 5B. Following the procedure of Example 4C, 50 g. of the lactam intermediate were transformed into 26 g. of 1,3-dihydro-7,8-dimethoxy-2H-3-benzazepine-2-thione. The thione intermediate (23.6 g.) was then transformed to the title product in the same manner as described in Example 4D. Formation of the salt in the usual way gave 17.15 g. of the title product, m.p. about 269-271°C. with decomposition.

Analysis: $C_{12}H_{16}N_2O_2 \cdot HCl$;
Calc.: C, 56.59; H, 5.94; N, 11.00; Cl, 13.92;
Found: C, 56.30; H, 5.93; N, 10.84; Cl, 13.82.

Example 7

2-Amino-7-ethoxy-8-methoxy-1H-3-benzazepine hydrochloride

Following the procedures of Example 4, 4-ethoxy-3-methoxyphenylacetic acid was transformed into 650 mg. of the title product, m.p. about 250-251°C.

Analysis: $C_{13}H_{16}N_2O_2 \cdot HCl$;
Calc.: C, 58.10; H, 6.38; N, 10.42; Cl, 13.19;
Found: C, 57.81; H, 6.28; N, 10.29; Cl, 12.89.

Example 8

2-Amino-7-methoxy-8-methyl-1H-3-benzazepine hydrochloride

Following the procedures of Example 4, 4-methoxy-3-methylphenylacetic acid was transformed into 2.2 g. of the title product, m.p. about 271.5-272.5°C. (recrystallized from acetonitrile).

Analysis: $C_{12}H_{14}N_2O \cdot HCl$;
Calc.: C, 60.38; H, 6.33; N, 11.74;
Found: C, 60.59; H, 6.51; N, 11.59.

Example 9

2-Amino-7,8-(methylenedioxy)-1H-3-benzazepine hydrochloride

The title product was prepared by converting 3,4-(methylenedioxy)phenylacetic acid to 10 g. of the lactam intermediate following the procedures of Examples 4A and 4B. The lactam intermediate was converted to the title product by first converting the lactam to 8.7 g. of the 2-ethoxy intermediate following the procedure of Example 5C. The 2-ethoxy intermediate was refluxed for three hours in a methanol solution of ammonium chloride. On evaporation of the solvent, 1N hydrochloric acid was added and the mixture stirred at room temperature. Filtration of the resulting solid and washing with ethanol and hexane provided 3.2 g. of the title compound. m.p. 250°C. (blackened), 270 (decomposed).

Analysis: $C_{11}H_{10}N_2O_2 \cdot HCl$;
Calc.: C, 55.36; H, 4.65;
Found: C, 55.81; H, 4.39.

## Example 10

2-Amino-8-nitro-1H-3-benzazepine

The intermediate 2-amino-1H-3-benzazepine was prepared according to the method of Johnson and Nasutavicus, Journal of Heterocyclic Chemistry, 2, 26 (1965). Twenty grams of this intermediate were added to 100 ml. of 90% nitric acid at about 0°C. The reaction was stirred at 0-10°C. for about three hours and for about two hours at room temperature. The solution was poured over ice and the resulting precipitate was filtered. After washing the precipitate with acetone, the precipitate was slurried in water and neutralized with saturated aqueous sodium carbonate solution. The desired product was recovered by filtration and dried to give 20 g. of the title product as an orange colored solid. The product decomposes at about 190-195°C. and melts at about 200-203°C.

Analysis: $C_{10}H_9N_3O_2$;
Calc.: C, 59.11; H, 4.46;
Found: C, 58.77; H, 4.39.

## Example 11

2-Methylamino-7,8-dimethoxy-1H-3-benzazepine

Two grams of 2-amino-7,8-dimethoxy-1H-3-benzazepine were slurried in 100 ml. of methanol. With stirring, 2.4 g. of methylamine hydrochloride were added. A complete solution was obtained followed by

precipitation of a white solid. After stirring for two hours the reaction was concentrated *in vacuo* to a white foam. The foam was dissolved in water and made basic to pH 8.5. The solution was extracted with ethyl acetate and the ethyl acetate was dried and evaporated *in vacuo* to an oil. Crystallization of the oil from acetone gave 360 mg. of the title product (dihydrate) as a white solid. m/e 232; m.p. 215-219°C. (decomposition).

Analysis: $C_{13}H_{16}N_2O_2 \cdot 2H_2O$;
Calc.: C, 58.09; H, 6.37; N, 10.43;
Found: C, 58.38; H, 6.82; N, 10.75.

## Example 12

2-Cyclopropylamino-7,8-dimethoxy-1H-3-benzazepine hydrochloride

A solution of 1.0 g. of 2-ethoxy-7,8-dimethoxy-1H-3-benzazepine, 0.23 g. of cyclopropylamine, and 4 ml. of acetic acid in 25 ml. of methanol was refluxed for 2.5 hours and then allowed to stir at room temperature overnight. The mixture was evaporated to dryness and the residue was taken up in 1N hydrochloric acid. The solution was extracted three times with chloroform. The aqueous solution was made basic with sodium carbonate and was extracted with chloroform. This chloroform extract was dried over sodium sulfate and evaporated to dryness giving a white foam. The hydrochloride salt was

formed which was recrystallized from acetone/ethanol to give 200 mg. of the title product as a white solid, m.p. about 249-250°C.

Analysis: $C_{15}H_{18}N_2O_2 \cdot HCl$;
Calc.: C, 61.12; H, 6.50; N, 9.50;
Found: C, 61.03; H, 6.50; N, 9.42.

Example 13

1-Methyl-2-amino-7,8-dimethoxy-1H-3-benzazepine hydrochloride

A. Preparation of 2,7,8-trimethoxy-1H-3-benzazepine.

A solution of 1,3-dihydro-7,8-dimethoxy-2H-3-benzazepine-2-one and 29.2 g. of trimethyloxonium tetrafluoroborate in 500 ml. of methylene chloride was stirred at room temperature for about three days. Four hundred milliliters of a 10% sodium carbonate solution were added to the reaction mixture and the layers were separated. The organic layer was dried over sodium sulfate and evaporated to dryness yielding 29.4 g. of the desired product as a tan solid, m.p. about 63-64°C.

B. Preparation of 1-methyl-2,7,8-trimethoxy-1H-3-benzazepine.

A solution of 6.6 g. of diisopropylamine in about 70 ml. of dry tetrahydrofuran was cooled to

about -78°C. and 40.6 ml. of a 1.6M solution of n-butyllithium were added to the solution. The solution was stirred at -78°C. for about 20 minutes at which time a solution of 12.0 g. of 2,7,8-trimethoxy-1H-3-benzazepine in about 120 ml. of tetrahydrofuran was added dropwise and the reaction was allowed to warm to room temperature while stirring over a period of about one hour. At this time 11.5 g. of methyl iodide were added and the solution was stirred at room temperature for three hours. The reaction mixture was quenched with about 300 ml. of a saturated sodium chloride solution, neutralized with 1N hydrochloric acid, and extracted into diethyl ether. The organic layer was dried over sodium sulfate and evaporated to dryness yielding 12.8 g. of a yellow-brown oil which solidified on trituration with hexanes. NMR analysis was consistent with the structure of the desired intermediate.

C. Preparation of 1-methyl-2-amino-7,8-dimethoxy-1H-3-benzazepine hydrochloride.

A solution of 5.0 g. of 1-methyl-2,7,8-trimethoxy-1H-3-benzazepine and 10.7 g. of ammonium acetate in 125 ml. of methanol was refluxed under a nitrogen atmosphere for three hours. After cooling the mixture was evaporated to dryness and the residue was dissolved in water. The pH was adjusted to 2 with 1N hydrochloric acid and the solution extracted with ethyl acetate. The aqueous layer was made basic with potassium carbonate

and was extracted with ethyl acetate. The ethyl acetate was dried over sodium sulfate and evaporated to dryness yielding 1.2 g. of a tan foam. The hydrochloride salt was formed by the addition of a saturated ethanolic hydrogen chloride solution to the free base dissolved in ethanol. Recrystallization from ethyl acetate/methanol yielded 0.9 g. of the title product as tan crystals, m.p. about 177-178°C.

Analysis: $C_{13}H_{16}N_2O_2 \cdot HCl$;
Calc.: C, 58.10; H, 6.38; N, 10.42;
Found: C, 57.84; H, 6.46; N, 10.20.

The title product was similarly prepared from the 2-ethoxy intermediate from Example 5C by the same procedure.

## Example 14

2-Amino-7,8-diethoxy-1H-3-benzazepine hydrochloride

A. Preparation of 1,3-dihydro-7,8-dihydroxy-2H-3-benzazepine-2-one.

To a solution of 29.16 g. of 1,3-dihydro-7,8-dimethoxy-2H-3-benzazepine-2-one in 800 ml. of methylene chloride cooled to approximately -78°C. with an external dry ice/acetone bath was added a solution of 100 g. of boron tribromide in 200 ml. of methylene chloride. The reaction was allowed to warm to room temperature and was stirred for about three days.

Twenty ml. of water were added to the reaction and after stirring for several hours the reaction was filtered. The precipitate was slurried in hot methanol and then allowed to cool. Filtration of the methanol gave 24.2 g. of the desired 7,8-dihydroxy intermediate, m.p. >260°C.

B. Preparation of 1,3-dihydro-7,8-diethoxy-2H-3-benzazepine-2-one.

A solution of 5.0 g. of 1,3-dihydro-7,8-dihydroxy-2H-3-benzazepine-2-one, 4.67 ml. of ethyl iodide, and 10.84 g. of potassium carbonate in acetone was allowed to reflux for about 24 hours. The solvent was removed by evaporation and the resulting residue was taken up in chloroform. The solution was extracted with 10% sodium hydroxide. Evaporation of the organic solvent afforded 2.96 g. of the desired 1,3-dihydro-7,8-diethoxy-2H-3-benzazepine-2-one, m.p. about 214-216°C.

C. Preparation of 2-amino-7,8-diethoxy-1H-3-benzazepine hydrochloride.

Following the procedure of Example 4C, 3.48 g. of 1,3-dihydro-7,8-diethoxy-2H-3-benzazepine-2-one were converted to 2.5 g. of the thiolactam intermediate. The thiolactam intermediate (250 mg.) was then converted into 233 mg. of the desired title product, m.p. 218-221°C. following the procedure of Example 4D. Mass spectral analysis showed the parent ion $M^+$ = 246.

## Example 15

2-Isopropylamino-7,8-dimethoxy-1H-3-benzazepine hydrochloride

Following the procedure of Example 12, 5.0 g. of 2-ethoxy-7,8-dimethoxy-1H-3-benzazepine, 3 ml. of isopropylamine, and 1 ml. of acetic acid were reacted in 100 ml. of methanol. After workup of the reaction mixture and formation of the hydrochloride salt, 4.0 g. of the title product were obtained, m.p. about 246-247°C.

Analysis: $C_{15}H_{20}N_2O_2 \cdot HCl$;
Calc.: C, 60.70; H, 7.13; N, 9.44;
Found: C, 60.49; H, 6.84; N, 9.26.

## Examples 16-22

Following the procedure of Example 11, the following N-substituted compounds of this invention were prepared from the corresponding 2-amino-benzazepine and the hydrohalide or acetate salt of the appropriate amine.

16. 2-Methylamino-1H-3-benzazepine, m.p. about 138-140°C., yield 2 g.

Analysis: $C_{11}H_{12}N_2$;
Calc.: C, 76.71; H, 7.02; N, 16.27;
Found: C, 76.54; H, 6.91; N, 16.56.

17. 2-Sec-butylamino-1H-3-benzazepine, m.p. about 124-126°C., yield 670 mg.

Analysis: $C_{14}H_{18}N_2$;
Calc.: C, 78.46; H, 8.47; N, 13.07;
Found: C, 78.67; H, 8.32; N, 12.82.

18. 2-Isobutylamino-1H-3-benzazepine, m.p. about 135-136°C. with decomposition, yield 550 mg.

Analysis: $C_{14}H_{18}N_2$;
Calc.: C, 78.46; H, 8.47; N, 13.07;
Found: C, 78.29; H, 8.63; N, 13.01.

19. 2-Cyclopropylamino-1H-3-benzazepine, m.p. about 159-160°C., yield 1.2 g.

Analysis: $C_{13}H_{14}N_2$;
Calc.: C, 78.75; H, 7.12; N, 14.13;
Found: C, 78.47; H, 6.91; N, 13.94.

20. 2-Allylamino-1H-3-benzazepine, m.p. about 118-120°C., yield 2.25 g.

Analysis: $C_{13}H_{14}N_2$;
Calc.: C, 78.75; H, 7.12; N, 14.13;
Found: C, 78.58; H, 7.00; N, 13.87.

21. 2-Isopropylamino-1H-3-benzazepine, m.p. about 161-162°C., yield 0.4 g.

Analysis: $C_{13}H_{16}N_2$;
Calc.: C, 77.96; H, 8.05; N, 13.99;
Found: C, 78.25; H, 8.07; N, 14.01.

22. 2-Ethylamino-7,8-dimethoxy-1H-3-benzazepine, m.p. about 89-92°C., yield 1.2 g.

Analysis: $C_{14}H_{18}N_2O_2 \cdot 0.33\ H_2O$;
Calc.: C, 66.64; H, 7.46; N, 11.10;
Found: C, 66.62; H, 7.03; N, 10.95.

Example 23

2-Allylamino-7,8-dimethoxy-1H-3-benzazepine

Following the procedure of Example 12, 1.0 g. of 2-ethoxy-7,8-dimethoxy-1H-3-benzazepine, 0.23 g. of allylamine, and 3 ml. of acetic acid in 25 ml. of methanol were reacted to give 150 mg. of the title product, m.p. about 211-212°C.

Analysis: $C_{15}H_{18}N_2O_2$;
Calc.: C, 61.12; H, 6.50; N, 9.50;
Found: C, 61.36; H, 6.73; N, 9.51.

Example 24

2-Amino-8-methyl-1H-3-benzazepine

Following the procedure of Example 4, 3-methylphenylacetic acid was transformed into 241.5 mg. of the title product. m.p. 223-225°C. The NMR and infrared spectra were consistent with the structure of the desired product.

## Example 25

2-Cyclopropylamino-7-ethoxy-8-methoxy-1H-3-benzazepine hydrochloride

A solution of 498 mg. of 1,3-dihydro-7-ethoxy-8-methoxy-2H-3-benzazepine-2-thione, 125.62 mg. of cyclopropylamine, and 5 ml. of triethylamine in 25 ml. of absolute ethanol was allowed to reflux for about 48 hours. The mixture was evaporated *in vacuo* and the residue was azeotroped with absolute alcohol three times. The residue was then dissolved in about 8 ml. of glacial acetic acid and the solution was cooled with an external ice bath. Gaseous hydrogen chloride was bubbled into the solution after which was added diethyl ether. The resulting precipitate was collected by filtration. After crystallization from ethanol and then from acetonitrile/water, 250 mg. of the title product were obtained, m.p. about 221°C.

Analysis: $C_{16}H_{20}N_2O_2 \cdot HCl$;
Calc.: C, 62.23; H, 6.85; N, 9.07; Cl, 11.48;
Found: C, 62.30; H, 7.06; N, 9.06; Cl, 11.29.

## Example 26

2-Amino-6,9-dimethyl-1H-3-benzazepine hydrochloride

Following the procedure of Examples 1B-1E, 2,5-dimethylbenzyl chloride was transformed into 50 mg. of the title product, m.p. >250°C.

Analysis: $C_{12}H_{14}N_2 \cdot HCl$;
Calc.: C, 64.71; H, 6.79; N, 12.58;
Found: C, 64.69; H, 6.96; N, 12.41.

Example 27

2-Amino-7-methoxy-8-hydroxy-1H-3-benzazepine hydrochloride

A slurry of 66.5 g. of aluminum chloride in 250 ml. of 1,2-dichloroethane was cooled to -20°C. with an external dry ice/acetone bath. With stirring, 43.4 g. of ethanethiol were added after which were added 25.45 g. of 2-amino-7,8-dimethoxy-1H-3-benzazepine hydrochloride. After stirring for two hours, the brown solution was added to water. Sodium chloride was added and the solution was concentrated to a slurry. The slurry was filtered and the filtrate was evaporated to dryness. The residue was slurried in hot acetic acid and filtered. Hydrogen chloride gas was bubbled through the acetic acid filtrate. After filtration of the solution, the acetic acid solution was concentrated *in vacuo* to approximately one-third of the original volume. Methanol crystallization of the precipitate obtained by filtration gave 3.7 g. of purified 2-amino-7-methoxy-8-hydroxy-1H-3-benzazepine hydrochloride. m.p. 260-262°C.

Analysis: $C_{11}H_{12}N_2O_2 \cdot HCl$;
Calc.: C, 54.89; H, 5.44; N, 11.64; O, 13.29;
Found: C, 55.15; H, 5.54; N, 11.51; O, 13.55.

Example 28

2-Amino-7-hydroxy-8-methoxy-1H-3-benzazepine hydrochloride

A solution of 2.54 g. of 2-amino-7,8-dimethoxy-1H-3-benzazepine hydrochloride and 1.7 g. of methionine in 20 equivalents of methanesulfonic acid was stirred at room temperature overnight. The reaction was poured into ice water and the pH was adjusted to 8-9. Filtration of the suspension afforded a tan precipitate which was dissolved in 1N hydrochloric acid. Stirring the solution at room temperature resulted in the precipitation of the title product which was isolated by filtration. m/e 240; m.p. 255-256°C. Yield 1.3 g.

Analysis: $C_{11}H_{12}N_2O_2 \cdot HCl$;
Calc.: C, 54.89; H, 5.44; N, 11.64; O, 13.29;
Found: C, 54.62; H, 5.67; N, 11.56; O, 13.33.

Example 29

2-Propargylamino-7,8-dimethoxy-1H-3-benzazepine hydrochloride

The title product was obtained from 0.8 g. of 2-ethoxy-7,8-dimethoxy-1H-3-benzazepine and 0.2 g. of propargylamine following the procedure of Example 12. The hydrochloride salt was crystallized from acetone/ethanol yielding 260 mg., m.p. about 213-214°C. The mass spectrum and NMR spectrum were consistent with the structure of the desired product.

## Example 30

2-Amino-1H-naphth[1,2-d]azepine hydrochloride

Following the procedures of Examples 1C-IE, 1-naphthylacetic acid was transformed into 40 mg. of the title product, m.p. decomposition >240°C.

Analysis: $C_{14}H_{12}N_2 \cdot HCl$;
Calc.: C, 68.71; H, 5.35; N, 11.45;
Found: C, 67.42; H, 5.30; N, 11.93.

## Example 31

2-Amino-1,7,8,9-tetrahydroindeno[5,6-d]-azepine hydrochloride

The title product was prepared from indan following the procedures of Example 1, m.p. about 256-257°C. with decomposition, yield 0.3 g.

Analysis: $C_{13}H_{14}N_2 \cdot HCl$;
Calc.: C, 66.52; H, 6.44; N, 11.93;
Found: C, 66.33; H, 6.44; N, 11.81.

## Example 32

2-Amino-6,9-dimethoxy-1H-3-benzazepine hydrochloride

Following the procedures of Examples 1C-1E, 2,5-dimethoxyphenylacetic acid was transformed into 0.22 g. of the title product, m.p. about 228-230°C. with decomposition.

Analysis: $C_{12}H_{14}N_2O_2 \cdot HCl$;
Calc.: C, 56.59; H, 5.94; N, 11.00;
Found: C, 56.44; H, 5.86; N, 10.89.

## Example 33

2-Amino-7-methyl-6,9-dimethoxy-1H-3-benzazepine hydrochloride

The title product was prepared from 2,5-dimethoxytoluene following the procedures of Example 1, m.p. about 224-225°C. with decomposition, yield 70 mg.

Analysis: $C_{13}H_{16}N_2O_2 \cdot HCl$;
Calc.: C, 58.10; H, 6.38; N, 10.42;
Found: C, 58.37; H, 6.50; N, 10.17.

## Examples 34 and 35

2-Amino-7,8,9,10-tetrahydro-1H-naphth[2,3-d]-azepine hydrochloride and 2-amino-8,9,10,11-tetra-hydro-1H-naphth[1,2-d]azepine hydrochloride

Following the procedure of Example 1A, 1,2,3,4-tetrahydronaphthalene was transformed into a

mixture of the corresponding 5- and 6-chloromethyl derivatives. This mixture was subjected to the procedures of Examples 1B-1E to provide the title compounds as a mixture. Fractional crystallization from ethyl acetate/ethanol gave the individual isomers.

2-Amino-7,8,9,10-tetrahydro-1H-naphth[2,3-d]-azepine hydrochloride, m.p. about 259-261°C. with decomposition. Mass spectral analysis gave the parent ion $M^+$ = 212. Yield 30 mg.

2-Amino-8,9,10,11-tetrahydro-1H-naphth[1,2-d]-azepine hydrochloride, m.p. about 238-240°C. with decomposition. Mass spectral analysis gave the parent ion $M^+$ = 212. Yield 25 mg.

## Example 36

2-Amino-8-fluoro-7-methoxy-1H-3-benzazepine hydrochloride

A. Preparation of N-(2,2-dimethoxyethyl)-3-fluoro-4-methoxyphenylacetamide.

To a solution of 30 g. of 3-fluoro-4-methoxyphenylacetic acid in 150 ml. of dichloromethane were added 26.3 g. of 1,1'-carbonyldiimidazole. A solution of 18.9 g. of aminoacetaldehyde dimethyl acetal in dichloromethane was slowly added. The mixture was stirred at room temperature for about 1 hour. The solution was then worked-up according to the procedure in Example 4A to yield 40.1 g. of the product.

B. Preparation of 1,3-dihydro-8-fluoro-7-methoxy-2H-3-benzazepine-2-one.

One g. of N-(2,2-dimethoxyethyl)-3-fluoro-4-methoxyphenylacetamide in 5 ml. of concentrated sulfuric acid was stirred in an ice bath and gradually allowed to warm to room temperature, stirred for 40 hours, poured onto ice, stirred and filtered. The white precipitate was crystallized from ethanol, affording 110 mg. of the product. m.p. about 234°C.

C. Preparation of 1,3-dihydro-8-fluoro-7-methoxy-2H-3-benzazepine-2-thione.

A solution of 1 g. of 1,3-dihydro-8-fluoro-7-methoxy-2H-3-benzazepine-2-one, 0.556 g. of phosphorous pentasulfide, 1.2 ml. of triethylamine and 25 ml. of acetonitrile was refluxed for 40 hours. After cooling, the reaction mixture was filtered, concentrated, dissolved in ethanol, concentrated, dissolved in acetonitrile and crystallized to yield 266 mg. of the product. m.p. about 222°C.

D. Preparation of 2-amino-8-fluoro-7-methoxy-1H-3-benzazepine hydrochloride.

A mixture of 317.5 mg. of 1,3-dihydro-8-fluoro-7-methoxy-2H-3-benzazepine-2-thione and 50 ml. of anhydrous ammonia was heated to 40°C. in a sealed bomb for three hours. After the ammonia was removed

by evaporation, 20 ml. of glacial acetic acid was added and the mixture cooled in an ice bath. Anhydrous hydrogen chloride gas was bubbled into the solution, diethyl ether was added and the mixture filtered. The precipitate was crystallized from hot acetonitrile to provide 83.4 mg. of the title product. m.p. about 260°C. (decomposition).

Example 37

2-Amino-8-ethoxy-7-methoxy-1H-3-benzazepine hydrochloride

Following the procedure of Example 4, 3-ethoxy-4-methoxybenzyl chloride was transformed into the title product, m.p. about 254-255°C.

Example 38

2-Amino-6,7,8-trimethoxy-1H-3-benzazepine hydrochloride

A. Preparation of 1,3-dihydro-6,7,8-tri-methoxy-2H-3-benzazepine-2-thione

Phosphorous pentasulfide, 11.8 g., was slurried in 100 ml. of acetonitrile and 28 ml. of triethylamine, and then heated on a steam bath until a solution formed. To the solution was added 12.5 g. of 1,3-dihydro-6,7,8-trimethoxy-2H-3-benzazepine-2-one (prepared by the process of Example 4) in one portion. The mixture was heated two hours and concentrated to dryness. The

residue was taken up in acetonitrile, water was added, the precipitate filtered and washed with ethanol and diethyl ether to yield 7.4 g. of the desired 1,3-dihydro-6,7,8-trimethoxy-2H-3-benzazepine-2-thione as an orange solid.

B. Preparation of 2-amino-6,7,8-trimethoxy-1H-3-benzazepine

1,3-Dihydro-6,7,8-trimethoxy-2H-3-benzazepine-2-thione, 7.0 g., was heated in methanol with 50 ml. of ammonia for 3 hours at 40°C. The mixture was concentrated and filtered to yield 2.3 g. of the title product as the free base. m/e 248, 233, 206, 191 and 175.

Analysis: $C_{13}H_{16}N_2O_3$;
Calc.: C, 62.89; H, 6.50; N, 11.28;
Found: C, 61.42; H, 5.69; N, 7.90.

C. Preparation of 2-amino-6,7,8-trimethoxy-1H-3-benzazepine hydrochloride

The product from Example B above was dissolved in acetic acid and hydrogen chloride gas bubbled through. The precipitate was filtered to yield 1.8 g. of the title product.

Analysis: $C_{13}H_{16}N_2O_3 \cdot HCl$
Calc.: C, 54.84; H, 6.02; N, 9.84;
Found: C, 54.61; H, 5.90; N, 9.62.

Example 39

2,8-Diamino-1H-3-benzazepine

2-Amino-8-nitro-1H-3-benzazepine, 4.06 g. (prepared as described in Example 10) was reduced with 0.5 g. of Raney nickel in 1 l. of methanol for one hour at room temperature. The mixture was concentrated to dryness. The residue was taken up in water, made basic and filtered to yield 1.2 g. of an amorphous solid of the title product. m/e 173, 145, 130.

The following formulation Examples employ as active compounds one of the compounds in Examples 1-39 or one of the other pharmaceutical compounds of formula I. Optionally, more than one active compound of formula I can be used.

Example 40

Hard gelatin capsules are prepared using the following ingredients:

| | per capsule |
|---|---|
| Active compound | 250 mg. |
| Starch dried | 200 mg. |
| Magnesium stearate | 10 mg. |
| Total | 460 mg. |

The above ingredients are mixed and filled into hard gelatin capsules in 460 mg. quantities.

Example 41

Capsules each containing 20 mg. of medicament are made as follows:

| | per capsule |
|---|---|
| Active ingredient | 20 mg. |
| Starch | 89 mg. |
| Microcrystalline cellulose | 89 mg. |
| Magnesium stearate | 2 mg. |
| Total | 200 mg. |

The active ingredient, cellulose, starch and magnesium stearate are blended, passed through a No. 45 mesh U.S. sieve and filled into hard gelatin capsules in 200 mg. quantities.

Example 42

Capsules each containing 100 mg. of active ingredient are made as follows:

| | per capsule |
|---|---|
| Active ingredient | 100 mg. |
| Polyoxyethylenesorbitan monooleate | 50 mcg. |
| Starch powder | 250 mg. |

The above ingredients are thoroughly mixed and are placed in an empty gelatin capsule.

Example 43

Tablets each containing 10 mg. of active ingredient are made up as follows:

| | per tablet |
|---|---|
| Active ingredient | 10 mg. |
| Starch | 45 mg. |
| Microcrystalline cellulose | 35 mg. |
| Polyvinylpyrrolidone (as 10% solution in water) | 4 mg. |
| Sodium carboxymethyl starch | 4.5 mg. |
| Magnesium stearate | 0.5 mg. |
| Talc | 1 mg. |
| Total | 100 mg. |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50-60°C. and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 100 mg.

## Example 44

A tablet formula is prepared using the ingredients below:

| | per tablet |
|---|---|
| Active compound | 250 mg. |
| Cellulose microcrystalline | 400 mg. |
| Silicon dioxide fumed | 10 mg. |
| Stearic acid | 5 mg. |
| Total | 665 mg. |

The components are blended and compressed to form tablets each weighing 665 mg.

## Example 45

Suppositories each containing 25 mg. of active ingredient are made as follows:

| | per suppository |
|---|---|
| Active ingredient | 25 mg. |
| Saturated fatty acid glycerides to | 2,000 mg. |

The active ingredient is passed through a No. 60 mesh U.S. sieve and suspended in the saturated fatty acid glycerides previously melted using the minimum heat necessary. The mixture is then poured into a suppository mold of nominal 2 g. capacity and allowed to cool.

Example 46

Suspensions each containing 5 mg. of medicament per 5 ml. dose are made as follows:

| | per 5 ml. of suspension |
|---|---|
| Active ingredient | 5 mg. |
| Sodium carboxymethyl cellulose | 50 mg. |
| Syrup | 1.25 ml. |
| Benzoic acid solution | 0.10 ml. |
| Flavor | q.v. |
| Color | q.v. |
| Purified water to | 5 ml. |

The medicament is passed through a No. 45 mesh U.S. sieve and mixed with the sodium carboxymethylcellulose and syrup to form a smooth paste. The benzoic acid solution, flavor and color is diluted with some of the water and added, with stirring. Sufficient water is then added to produce the required volume.

Example 47

An aerosol solution is prepared containing the following components:

| | Weight % |
|---|---|
| Active ingredient | 0.25 |
| Ethanol | 29.75 |
| Propellant 22 (Chlorodifluoromethane) | 70 |

The active compound of formula I is mixed with ethanol and the mixture added to a portion of the propellant, cooled to -30°C. and transferred to a filling device. The required amount is then fed to a stainless steel container and diluted further with the remaining amount of propellant. The valve units are then fitted to the container.

The compounds of formula I, either as such or in the form of an acid addition salt thereof, are useful in lowering blood pressure. Although they demonstrate this useful activity in the laboratory in both normotensive and spontaneously hypertensive mammals, the compounds would be employed to lower blood pressure of mammals having an elevated blood pressure. Thus, this invention also provides a method of lowering blood pressure in a mammal having an elevated blood pressure and in need of treatment, employing a hypotensive dose of a compound of formula I. The pharmaceutically acceptable salts of the compounds of formula I are, of course, equally useful in treating hypertension.

Certain compounds of formula I were examined as to their pharmacodynamic effects in the following test system.

Determination of Antihypertensive Activity

Spontaneously hypertensive male rats were anesthetized with methoxyflurane and α-chloralose (120 mg./kg., i.v.). An aqueous solution of the compound of formula I to be tested was administered i.p. to the animal following a 15-20 minute equilibrium period. Systemic arterial blood pressure was measured from the right carotid artery via a Statham P23 ID pressure transducer and recorded on a Model R411 Beckman Dynograph recorder. Heart rate was recorded with a Beckman 9857B cardiotachometer triggered by the pulse pressure. Mean arterial pressure was calculated as the diastolic pressure plus one third of the pulse pressure. Antihypertensive responses were measured at the maximal reduction in blood pressure occurring within 30 minutes after drug administration as compared to the baseline measurements taken prior to administration of the compound. The effect on heart rate was determined at the same time the maximum reduction in blood pressure was seen and was also compared with the baseline heart rate prior to compound administration.

The results in Table I are reported as the maximal reduction in blood pressure and the corresponding effect on heart rate for certain compounds of formula I. The results are the average of two or more experiments.

Table I

Effect of Compounds of Formula I on blood pressure and heart rate in spontaneously hypertensive rats*

| Compound of Example No. | Effect on blood pressure** | Effect on heart rate*** |
|---|---|---|
| 1 | -24.0% | ND**** |
| 2 | -46.7% | -21.7% |
| 3 | -31.0% | -20.0% |
| 4 | -29.5% | -23.5% |
| 5 | -43.0% | -21.0% |
| 6 | -56.0% | -15.0% |
| 7 | -36.0% | -3.0% |
| 8 | -54.0% | -31.0% |
| 9 | -19.0% | -11.0% |
| 10 | -55.0% | -22.0% |
| 11 | -36.0% | -7.0% |
| 12 | -25.0% | -8.0% |
| 13 | -22.0% | -2.0% |
| 14 | -18.0% | -8.0% |
| 15 | -10.0% | -3.0% |
| 16 | -8.0% | -9.0% |
| 17 | -22.0% | -13.0% |
| 18 | -17.0% | -7.0% |
| 19 | -20.0% | -8.0% |
| 20 | -20.0% | -15.0% |
| 21 | -16.0% | -6.0% |
| 22 | -13.0% | 2.0% |
| 23 | -15.0% | -5.0% |
| 24 | -35.0% | -22.0% |
| 25 | -9.0% | -6.0% |
| 26 | -44.0% | ND |

Table I continued

| Compound of Example No. | Effect on blood pressure** | Effect on heart rate*** |
|---|---|---|
| 27 | -29.0% | -12.0% |
| 28 | -11.0% | 0.0% |
| 29 | -29.0% | -11.0% |
| 30 | -37.0% | -6.0% |
| 31 | -40.0% | -16.0% |
| 32 | -32.0% | -13.0% |
| 33 | -47.0% | -43.0% |
| 34 | -30.0% | -15.0% |
| 35 | -33.0% | -12.0% |
| 36 | -31.0% | -8.0% |
| 37 | -48.0% | -23.0% |

*compound given i.p. in an aqueous solution at a dose of 10 mg./kg. 15-20 minutes after anesthetization.

**maximal reduction in blood pressure within 30 minutes of compound administration compared with pre-treatment baseline values. Minus figures indicate a reduction in blood pressure.

***effect on heart rate at the time of maximum reduction in blood pressure as compared to pre-treatment baseline values. Minus figures indicate a decrease in heart rate.

****not determined.

CLAIMS

1. A compound of the formula

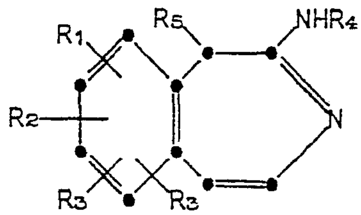

I

or the pharmaceutically acceptable salts thereof, wherein

each $R_1$ and $R_2$ is independently hydrogen, hydroxy, halo, $C_1$-$C_3$ alkyl, nitro, amino, mono- or di-$C_1$-$C_3$ alkylamino, trifluoromethyl, nitrile, amide, acetyl, or $C_1$-$C_3$ alkoxy, or when taken together form a methylenedioxy group or when taken together with the benzene ring to which they are attached form a naphthalene, tetrahydronaphthalene, dihydrobenzopyran, dihydrobenzofuran, or indan system;

each $R_3$ is independently hydrogen, halo, $C_1$-$C_3$ alkyl, or $C_1$-$C_3$ alkoxy;

$R_4$ is hydrogen, $C_1$-$C_4$ alkyl, allyl, propargyl, or cyclopropyl;

$R_5$ is hydrogen or methyl;

with the proviso that $R_1$, $R_2$, $R_3$, $R_4$ and $R_5$ may not all be hydrogen at the same time, and with the further proviso that when $R_3$ is hydrogen and one of $R_1$ and $R_2$ is hydroxy, the other of $R_1$ and $R_2$ may not be hydrogen or hydroxy.

2. A compound of claim 1 wherein each of $R_1$ and $R_2$ is independently hydrogen, hydroxy, halo, $C_1$-$C_3$ alkyl, nitro, amino, mono- or di-$C_1$-$C_3$ alkylamino, trifluoromethyl, or $C_1$-$C_3$ alkoxy, or when taken together form a methylenedioxy group or when taken together with the benzene ring to which they are attached form a naphthalene, tetrahydronaphthalene, or indan system;

one $R_3$ is hydrogen and the other $R_3$ is hydrogen, $C_1$-$C_3$ alkyl, or $C_1$-$C_3$ alkoxy;

$R_4$ is hydrogen, $C_1$-$C_3$ alkyl, allyl, propargyl, or cyclopropyl; and

$R_5$ is defined as in claim 1.

3. A compound of claim 1 wherein $R_3$, $R_4$, and $R_5$ are each hydrogen.

4. A compound of claim 1, 2 or 3 wherein $R_1$ is $C_1$-$C_3$ alkoxy.

5. A compound of claim 1, 2 or 3 wherein $R_1$ is $C_1$-$C_3$ alkyl.

6. A compound of claim 4 or 5 wherein $R_2$ is $C_1$-$C_3$ alkoxy.

7. A compound of claim 4 or 5 wherein $R_2$ is $C_1$-$C_3$ alkyl.

8. Any one of the following compounds, or a pharmaceutically-acceptable salt thereof:

2-amino-7,8-dimethoxy-1H-3-benzazepine
2-amino-7,8-dimethyl-1H-3-benzazepine
2-amino-6,9-dimethyl-1H-3-benzazepine
2-amino-7-methoxy-8-methyl-1H-3-benzazepine
2-amino-7-methyl-6,9-dimethoxy-1H-3-benzazepine
2-amino-1,7,8,9-tetrahydroindeno[5,6-d]azepine

2-amino-7-ethoxy-8-methoxy-1H-3-benzazepine
2-amino-8-ethoxy-7-methoxy-1H-3-benzazepine
2-amino-8,9,10,11-tetrahydro-1H-naph[1,2-d]-azepine

9. The compound of claim 1, 6 or 8 which is 2-amino-7,8-dimethoxy-1H-3-benzazepine hydrochloride.

10. A pharmaceutical formulation comprising as active ingredient one or more of the benzazepine derivatives of formula I, or a pharmaceutically-acceptable salt thereof, as claimed in any one of claims 1 to 9, associated with one or more pharmaceutically-acceptable carriers, diluents or excipients therefor.

11. A pharmaceutical formulation as claimed in claim 10 which is in the form of a tablet, capsule, or an i.v. or nasal solution or suspension.

12. A process for preparing a benzazepine derivative of any one of claims 1 to 9 which comprises:

(a) heating a compound of the formula

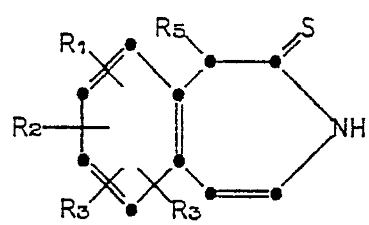

V

wherein $R_1$, $R_2$, $R_3$ and $R_5$ are defined as in claim 1, with $R_4NH_2$ or its salt wherein $R_4$ as defined above, optionally in the presence of an alcohol or acetonitrile; or

(b) heating a compound of the formula

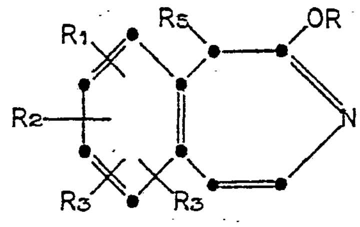

VI

wherein $R_1$, $R_2$, $R_3$ and $R_5$ are as defined in claim 1, and R is $C_1$-$C_6$ alkyl, with an acid salt of $R_4NH_2$ where $R_4$ is defined as in claim 1, in a non-reactive solvent; or

(c) reacting a compound of the formula

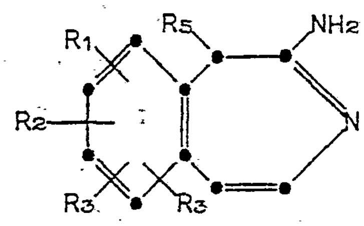

Ia

wherein $R_1$, $R_2$, $R_3$ and $R_5$ are defined as in claim 1, with $R_4'NH_2$ salt wherein $R_4'$ is $C_1$-$C_4$ alkyl, allyl, propargyl, or cyclopropyl, in an alcohol; or

(d) de-etherifying a compound of the formula

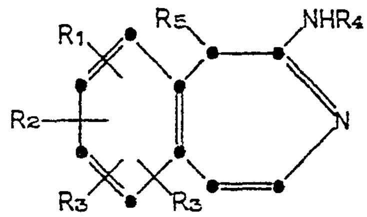

Ib

wherein $R_4$ and $R_5$ are defined as in claim 1, and at least one of $R_1$, $R_2$ and $R_3$ is $C_1$-$C_3$ alkoxy, with methanesulfonic acid/methionine, ethanethiol/aluminum chloride in the presence of 1,2-dichloroethane, $PBr_3$ in a halogenated hydrocarbon solvent, or HBr in glacial acetic acid; or

(e) nitrating a compound of the formula

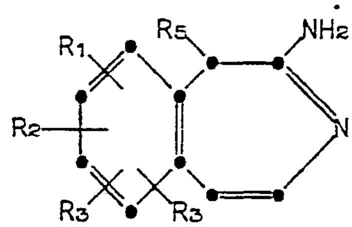

Ic

wherein $R_3$ and $R_5$ are defined as in claim 1, and one of $R_1$ and $R_2$ is hydrogen, with nitric acid;

optionally reducing the $NO_2$ group to provide the compounds of formula I wherein one of $R_1$ and $R_2$ is amino; and

optionally alkylating the $NH_2$ group of $R_1$ or $R_2$ to provide the compounds of formula I wherein one of $R_1$ and $R_2$ is mono- or di-$C_1$-$C_3$ alkylamino; or

(f) cleaving a compound of the formula

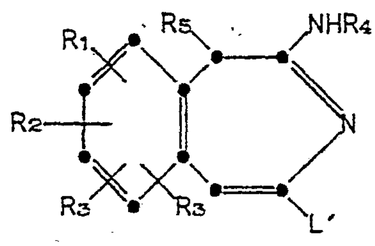

Id .

wherein $R_4$ and $R_5$ are defined as in claim 1, and L' is a leaving group, with a hydrogenation agent; or

(g) alkylating a compound of the formula

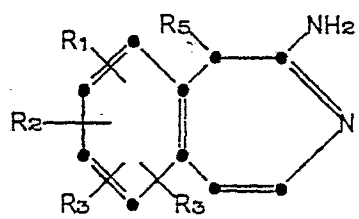

Ie

wherein $R_3$ and $R_5$ are defined as in claim 1, one or both of $R_1$ and $R_2$ are hydroxy, with alkyling agent, optionally in the presence of a base; or

(h) cyclizing a compound of the formula

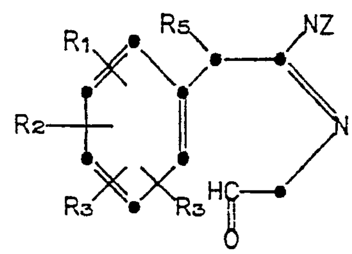

If

wherein $R_1$, $R_2$, $R_3$ and $R_5$ are defined as in claim 1, and Z is a protecting group, with an acid; or

(i) cleaving a protecting group from a compound of the formula

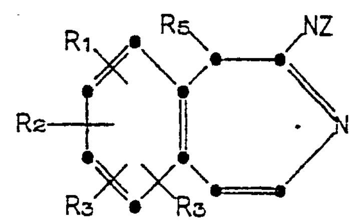

Ig

wherein $R_1$, $R_2$, $R_3$ and $R_5$ are defined as in claim 1, Z is a protecting group, with acid, base, a hydrogenation agent or water and $PdCl_2$; or

(j) reacting a compound of the formula

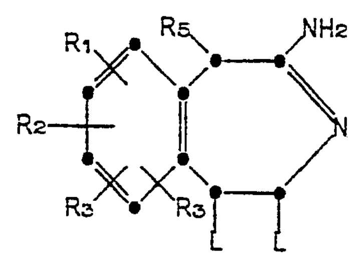

Ih

wherein $R_1$, $R_2$, $R_3$ and $R_5$ are defined as in claim 1, and at least one L is hydrogen and the other L is hydrogen or a leaving group, with, when L is other than hydrogen, an acid catalyst, at reflux temperature or with, when L both terms are hydrogen, dichlorodicyanobenzoquinone, $MnO_2$ or Pd; to introduce a $\Delta^4$ double bond; or

(k) cleaving a compound of the formula

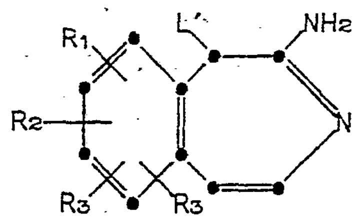

Ii

where $R_1$, $R_2$ and $R_3$ are defined as in claim 1, and L' is a leaving group, with a hydrogenating agent, in the presence of a base; or

(l) optionally salifying a product of any of the preceding steps by conventional methods.

13. A benzazepine derivative of formula I, or a pharmaceutically-acceptable salt thereof, as claimed in any one of claims 1 to 9, for use as an antihypertensive agent in mammals.

14. The use according to claim 13 wherein the compound of formula I is 2-amino-7,8-dimethoxy-1H-3-benzazepine, or a pharmaceutically-acceptable salt thereof.

15. A compound of the formula

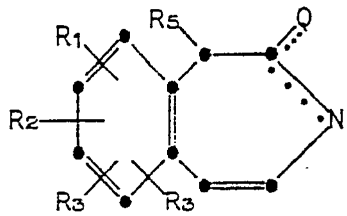

VII

wherein $R_1$, $R_2$, $R_3$ and $R_5$ are defined as in claim 1 for formula I, Q is halo, -O-($C_1$-$C_3$ alkyl), -S-($C_1$-$C_3$ alkyl), -SO-($C_1$-$C_3$ alkyl), -$SO_2$-($C_1$-$C_3$ alkyl), =O or =S, and the dotted line represents the presence of a double bond which is only exo to the ring when Q is =S or =O, or salts thereof.

16. A compound of claim 15 wherein Q is ethoxy or methoxy.

17. A compound of claim 15 wherein Q is =S.

## CLAIMS

1. A process for preparing a compound of the formula

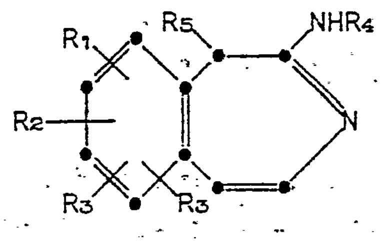

I

or the pharmaceutically acceptable salts thereof, wherein

each $R_1$ and $R_2$ is independently hydrogen, hydroxy, halo, $C_1$-$C_3$ alkyl, nitro, amino, mono- or di-$C_1$-$C_3$ alkylamino, trifluoromethyl, nitrile, amide, acetyl, or $C_1$-$C_3$ alkoxy, or when taken together form a methylenedioxy group or when taken together with the benzene ring to which they are attached form a naphthalene, tetrahydronaphthalene, dihydrobenzopyran, dihydrobenzofuran, or indan system;

each $R_3$ is independently hydrogen, halo, $C_1$-$C_3$ alkyl, or $C_1$-$C_3$ alkoxy;

$R_4$ is hydrogen, $C_1$-$C_4$ alkyl, allyl, propargyl, or cyclopropyl;

$R_5$ is hydrogen or methyl;

with the proviso that $R_1$, $R_2$, $R_3$, $R_4$ and $R_5$ may not all be hydrogen at the same time, and with the further proviso that when $R_3$ is hydrogen and one of $R_1$ and $R_2$ is hydroxy, the other of $R_1$ and $R_2$ may not be hydrogen or hydroxy; which comprises:

(a) heating a compound of the formula

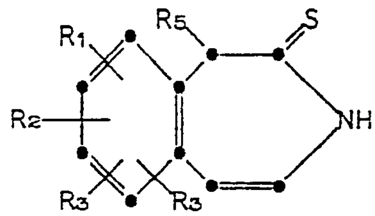

V

wherein $R_1$, $R_2$, $R_3$ and $R_5$ are defined as above, with $R_4NH_2$ or its salt wherein $R_4$ as defined above, optionally in the presence of an alcohol or acetonitrile; or

(b) heating a compound of the formula

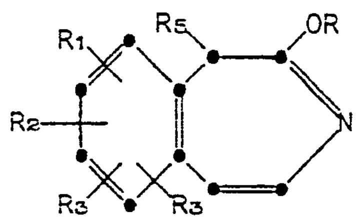

VI

wherein $R_1$, $R_2$, $R_3$ and $R_5$ are as defined above, and R is $C_1$-$C_6$ alkyl, with an acid salt of $R_4NH_2$ where $R_4$ is defined as in claim 1, in a non-reactive solvent; or

(c) reacting a compound of the formula

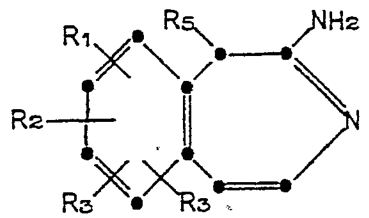

Ia

wherein $R_1$, $R_2$, $R_3$ and $R_5$ are defined as above, with $R_4'NH_2$ salt wherein $R_4'$ is $C_1$-$C_4$ alkyl, allyl, propargyl, or cyclopropyl, in an alcohol; or

(d) de-etherifying a compound of the formula

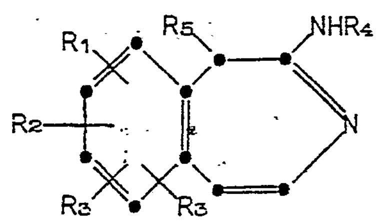

Ib

wherein $R_4$ and $R_5$ are defined as above, and at least one of $R_1$, $R_2$ and $R_3$ is $C_1$-$C_3$ alkoxy, with methanesulfonic acid/methionine, ethanethiol/aluminum chloride in the presence of 1,2-dichloroethane, $PBr_3$ in a halogenated hydrocarbon solvent, or HBr in glacial acetic acid; or

(e) nitrating a compound of the formula

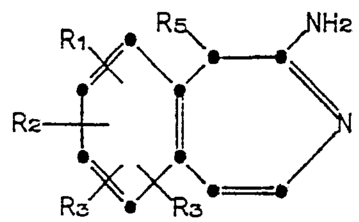

Ic

wherein $R_3$ and $R_5$ are defined as above, and one of $R_1$ and $R_2$ is hydrogen, with nitric acid;

optionally reducing the $NO_2$ group to provide the compounds of formula I wherein one of $R_1$ and $R_2$ is amino; and

optionally alkylating the $NH_2$ group of $R_1$ or $R_2$ to provide the compounds of formula I wherein one of $R_1$ and $R_2$ is mono- or di-$C_1$-$C_3$ alkylamino; or

(f) cleaving a compound of the formula

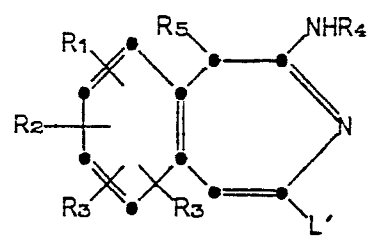

Id

wherein $R_4$ and $R_5$ are defined as above, and L' is a leaving group, with a hydrogenation agent; or

(g) alkylating a compound of the formula

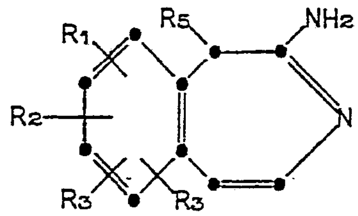

Ie

wherein $R_3$ and $R_5$ are defined as above, one or both of $R_1$ and $R_2$ are hydroxy, with alkyling agent, optionally in the presence of a base; or

(h) cyclizating a compound of the formula

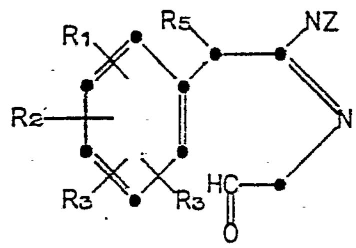

If

wherein $R_1$, $R_2$, $R_3$ and $R_5$ are defined as above, and Z is a protecting group, with an acid; or

(i) cleaving a protecting group from a compound of the formula

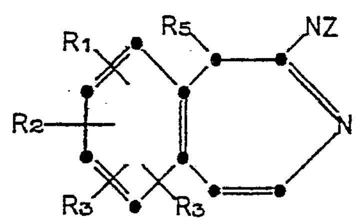

Ig

wherein $R_1$, $R_2$, $R_3$ and $R_5$ are defined as above, Z is a protecting group, with acid, base, a hydrogenation agent or water and $PdCl_2$; or

(j) reacting a compound of the formula

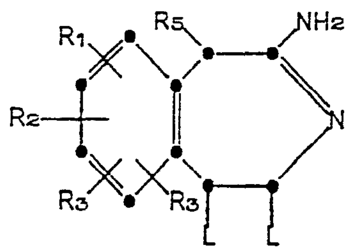

Ih

wherein $R_1$, $R_2$, $R_3$ and $R_5$ are defined as above, and at least one L is hydrogen and the other L is hydrogen or a leaving group, with, when L is other than hydrogen, an acid catalyst, at reflux temperature or with, when L both terms are hydrogen, dichlorodicyanobenzoquinone, $MnO_2$ or Pd; to introduce a $\Delta^4$ double bond; or

(k) cleaving a compound of the formula

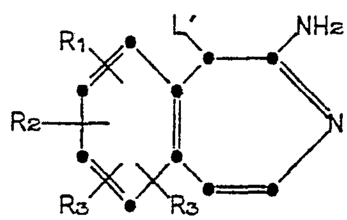

Ii

where $R_1$, $R_2$ and $R_3$ are defined as above, and L' is a leaving group, with a hydrogenating agent, in the presence of a base; or

(l) optionally salifying a product of any of the preceding steps by conventional methods.

2. A process of claim 1 wherein each of $R_1$ and $R_2$ is independently hydrogen, hydroxy, halo, $C_1$-$C_3$ alkyl, nitro, amino, mono- or di-$C_1$-$C_3$ alkylamino, trifluoromethyl, or $C_1$-$C_3$ alkoxy, or when taken together form a methylenedioxy group or when taken together with the benzene ring to which they are attached form a naphthalene, tetrahydronaphthalene, or indan system;

one $R_3$ is hydrogen and the other $R_3$ is hydrogen, $C_1$-$C_3$ alkyl, or $C_1$-$C_3$ alkoxy;

$R_4$ is hydrogen, $C_1$-$C_3$ alkyl, allyl, propargyl, or cyclopropyl; and

$R_5$ is defined as in claim 1.

3. The process of claim 1 (b) for preparing 2-amino-7,8-dimethoxy-1H-3-benzazepine which comprises reacting 2-ethoxy-7,8-dimethoxy-1H-3-benzazepine with ammonium chloride in methanol.

4. The process of claim 1 (a) for preparing 2-amino-7,8-dimethoxy-1H-3-benzazepine which comprises reacting 1,3-dihydro-7,8-dimethoxy-2H-3-benzazepine-2-thione with anhydrous ammonia.

5. The process of claim 1 (b) for preparing 2-amino-7,8-dimethyl-1H-3-benzazepine which comprises reacting 2-ethoxy-7,8-dimethyl-1H-3-benzazepine with ammonium acetate in methanol.

6. The process of claim 1 (b) for preparing 2-amino-6,9-dimethyl-1H-3-benzazepine which comprises reacting 2-ethoxy-6,9-dimethyl-1H-3-benzazepine with ammonium acetate in methanol.

7. The process of claim 1 (a) for preparing 2-amino-7-methoxy-8-methyl-1H-3-benzazepine which comprises reacting 1,3-dihydro-7-methoxy-8-methyl-2H-3-benzazepine-2-thione with anhydrous ammonia.

8. The process of claim 1 (b) for preparing 2-amino-7-methyl-6,9-dimethoxy-1H-3-benzazepine which comprises reacting 2-ethoxy-7-methyl-6,9-dimethoxy-1H-3-benzazepine with ammonium acetate in methanol.

9. The process of claim 1 (b) for preparing 2-amino-1,7,8,9-tetrahydroindeno[5,6-d]azepine which comprises reacting 2-ethoxy-1,7,8,9-tetrahydroindeno-[5,6-d]azepine with ammonium acetate in methanol.

10. The process of claim 1 (a) for preparing 2-amino-7-ethoxy-8-methoxy-1H-3-benzazepine which comprises reacting 1,3-dihydro-7-ethoxy-8-methoxy-2H-3-benzazepine-2-thione with anhydrous ammonia.

11. The process of claim 1 (a) for preparing 2-amino-8-ethoxy-7-methoxy-1H-3-benzazepine which comprises reacting 2-amino-8-ethoxy-7-methoxy-2H-3-benzazepine-2-thione with anhydrous ammonia.

12. The process of claim 1 (b) for preparing 2-amino-8,9,10,11-tetrahydro-1H-naph[1,2-d]azepine which comprises reacting 2-ethoxy-8,9,10,11-tetrahydro-1H-naph[1,2-d]azepine with ammonium acetate in methanol.